# EUROPEAN PATENT SPECIFICATION

(11) **EP 0 946 557 B1**
(45) Date of publication and mention of the grant of the patent: **11.01.2006**
(21) Application number: 97947567.0
(22) Date of filing: 19.11.1997
(51) Int. Cl.: C07D 473/06, A61K 31/52, G01N 33/06, G01N 33/60

(54) **A-1-ADENOSINE RECEPTOR ANTAGONISTS**
A-1-ADENOSIN REZEPTOR ANTAGONISTE
ANTAGONISTES DU RECEPTEUR A-1-D'ADENOSINE

(30) Priority: 19.11.1996 US 753048
(43) Date of publication of application: 06.10.1999
(73) Proprietor: Endacea, Inc., Raleigh, NC 27609-5750 (US)
(72) Inventor: NEELY, Constance, F., Raleigh, NC 27615 (US)
(74) Representative: Harrison Goddard Foote
(86) International application number: PCT/US1997/021045
(87) International publication number: WO 1998/022465

(56) References cited:
- EP-A- 0 501 379
- EP-A- 0 503 563
- EP-A- 0 764 647
- BE-A- 636 828
- US-A- 5 248 770
- J. MED. CHEM., vol. 35, no. 3, 1992, pages 407-422,

## Description

### Field and Background of the Invention

The present invention relates to novel compounds useful as A₁ adenosine receptor antagonists.

Adenosine receptors are involved in a vast number of peripheral and central regulatory mechanisms such as, for example, vasodilation, cardiac depression, inhibition of lipolysis, inhibition of insulin release and potentiation glucagon release in the pancreas, and inhibition of neurotransmitter release from nerve endings.

In general, adenosine receptors can be divided into two main classes. A₁ receptors which can inhibit, and A₂ receptors which can stimulate adenylate cyclase activity. One of the best known classes of adenosine receptor antagonists are the xanthines which include caffeine and theophylline. See e.g., Müller et al., *J. Med. Chem*. **33:** 2822-2828 (1990). In general, many of these antagonists often suffer from poor water solubility, and low potency or lack of selectivity for adenosine receptors. Additionally, selective analogues of adenosine receptor antagonists have been developed through the "functionalized congener" approach. Analogues of adenosine receptor ligands bearing functionalized chains have been synthesized and attached covalently to various organic moieties such as amines and peptides. Attachment of the polar groups to xanthine congeners has been found to increase water solubility. Nonetheless, such developments have yet to fully address problems associated with potency and selectivity. More recently Jacobson et al. *J. Med. Chem.* **35**: 408-422 (1992) has proposed various derivatives of adenosine and theophylline for use as receptor antagonists. The article discloses that hydrophobic substituents are able to potentially enhance affinity. However, it is also acknowledged that such substituents may result in a decrease in solubility thus rendering the antagonists less soluble *in vivo*. In confronting these problems, Jacobson et al. indicates that a dipropyl substitution at the 1 and 3 positions of theophylline allows desirable affinity at A₁ receptors. It is also stated that substitutions at the 7-position are typically not favorable.

Various other pharmacological applications of xanthine derivatives are reported in the prior art. Thus, for example, BE-A-636828 discloses the use of theophylline derivatives and pharmacologically acceptable salts thereof as choleretic agents, whilst EP-A-501379 teaches xanthine compounds substituted with adamantyl and either allyl or propargyl groups, and the diuretic and renal-protecting effects of these compounds. In addition, EP-A-503563 is concerned with certain substituted phenylalkyl xanthine derivaties, and their use as selective A1-adenosine receptor antagonists which are useful in the treatment of patients suffering from Alzheimer's disease, congestive heart failure or pulmonary bronchoconstriction, and EP-A-764647 reports the synthesis of xanthene derivatives substituted with benzylacetic acid moieties and their use as medicaments, in particular as antiatherosclerotic medicaments.

However, nowhere in the prior art is there reported the use of such xanthine derivatives in assay type-probes, and this was the primary concern of the present inventors. It is an object of the present invention to therefore provide compounds useful as A₁ adenosine receptor antagonists which display high potency and affinity levels, along with water solubility, and which are useful in assay-type probes.

### Summary of the Invention

In one aspect, the present invention provides an assay-type probe comprising
(1) a compound of the general formula: 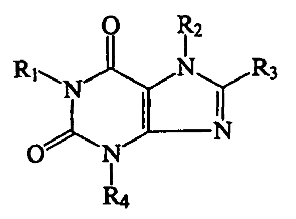
   wherein R₁ is selected from the group consisting of C₁-C₈ alkyl;
   R₂ is of the formula: 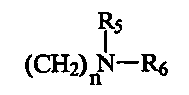
   wherein n is an integer ranging from 1 to 8; R₅ is H or CH₃(CH₂)ₚ, wherein p is an integer ranging from 1 to 7; and R₆ is H, (CH₂)ₘH, or (CH₂)ₘOH, wherein m is an integer ranging from 1 to 8;
   R₃ is:

   ―(CH₂)_{q}C₆H₄―R₇

   wherein q is an integer ranging from 1 to 8; wherein R₇ is selected from the group consisting of H, OH, NH₂, R₉COOH, wherein R₉ is an alkylene or alkenylene group having 1 to 8 carbon atoms, and (CH₂)ₜOH, wherein t is an integer ranging from 1 to 8; and
   R₄ is of the formula: 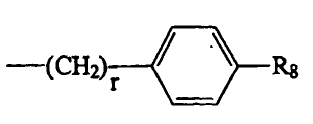
   wherein R₈ is selected from the group consisting of H; OH; (CH₂)_{f}NH₂ wherein f is selected from the group consisting of 0 and an integer ranging from 1 to 8; (CH₂)ₛOH, wherein s is an integer ranging from 1 to 8; and R₁₀COOH, wherein R₁₀ is an alkylene or alkenylene group having 1 to 8 carbon atoms; and r is an integer ranging from 1 to 8; and
(2) a radioactive or non-radioactive material, wherein the assay-type probe is marked or conjugated with the radioactive or non-radioactive material wherein when said compound is labelled with a non-radioactive material, a spacer component is present thereon wherein the spacer component has a functionality which bonds to the amine, hydroxyl or carboxy functionality present on the R₆, R₇ or R₈ substituent of said compound.

In a second aspect, the invention provides an assay-type probe which comprises a pharmaceutically acceptable salt of the above compound and a radioactive or non-radioactive material.

### Detailed Description of the Invention

The present invention will now be described more fully hereinafter, in which preferred embodiments of the invention are shown. This invention may, however, be embodied in different forms and should not be construed as limited to the embodiments set forth herein. Rather, these embodiments are provided so that this disclosure will be thorough and complete, and will fully convey the scope of the invention to those skilled in the art.

The present invention is directed to an assay type probe comprising:
(1) a compound of the formula (I): 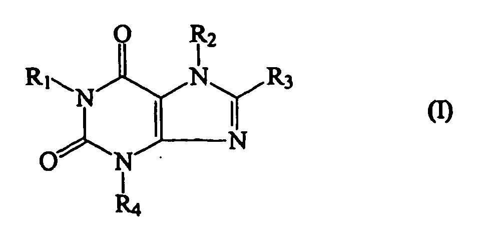
   R₁ is selected from the group consisting of C₁-C₈ alkyl, preferably C₁ to C₄ alkyl. For the purposes of the invention, R₁ is more preferably C₁ or C₃ alkyl, and is most preferably C₃ alkyl.
   R₂ is of the formula: 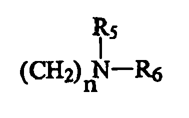
   wherein n is an integer ranging from 1 to 8, more preferably 1 to 4; R₅ is H or CH₃(CH₂)ₚ, wherein p is an integer ranging from 1 to 7, more preferably 1 to 4; and R₆ is H, (CH₂)ₘH, or (CH₂)ₘOH, wherein m is an integer ranging from 1 to 8, more preferably 1 to 4.
   R₃ is:

   -(CH₂)_{q}C₆H₄-R₇

   wherein q is an integer ranging from 1 to 8; and wherein R₇ is selected from the group consisting of H, OH, NH₂, R₉COOH, wherein R₉ is an alkylene or alkenylene group having 1 to 8 carbon atoms, and (CH₂)ₜOH, wherein t is an integer ranging from 1 to 8. The alkylene or alkenylene groups may be substituted or unsubstituted. R₉ is preferably CH=CH.
   R₄ is of the formula: 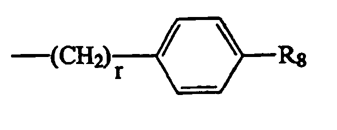
   wherein R₈ is selected from the group consisting of H; OH; (CH₂)_{f}NH₂, wherein f is selected from the group consisting vf 0 and an integer ranging from 1 to 8; (CH₂)ₛOH, wherein s is an integer ranging from 1 to 8, more preferably 1 to 4; and R₁₀COOH, wherein R₁₀ is an alkylene or alkenylene group having 1 to 8 carbon atoms; and r is an integer ranging from 1 to 8, more preferably 1 to 4. In the above, R₉ and R₁₀ are preferably CH=CH; and
(2) a radioactive or non-radioactive material, wherein the assay-type probe is marked or conjugated with the radioactive or non-radioactive material wherein when said compound is labelled with a non-radioactive material, a spacer component is present thereon wherein the spacer component has a functionality which bonds to the amine, hydroxyl or carboxy functionality present on the R₆, R₇ or R₈ substituent of said compound.

The invention may be illustrated below with respect to preferred embodiments. In these embodiments, R₃ is of the formula:

-(CH₂)_{q}C₆H₄-R₇

In one preferred embodiment, R₁ is C₃ alkyl; R₅ is CH₃ (CH₂)ₚ wherein p is 1; R₆ is (CH₂)ₘOH wherein m is 2; R₇ is H; R₈ is NH₂; f is 0; n is 2; m is 2; q is 1; and r is 2.

In another preferred embodiment, R₁ is C₃ alkyl; R₅ is CH₃ (CH₂)ₚ wherein p is 1; R₆ is H; R₇ is NH₂; R₈ is NH₂; f is 0; n is 2; q is 1; and r is 2.

In another preferred embodiment, R₁ is C₃ alkyl; R₅ is CH₃ (CH₂)ₚ wherein p is 1; R₆ is H; R₇ is H; R₈ is NH₂; f is 0; n is 2; q is 1; and r is 2.

In another preferred embodiment, R₁ is C₃ alkyl; R₅ is CH₃ (CH₂)ₚ wherein p is 1; R₆ is H; R₇ is H; R₈ is selected from the group consisting of (CH₂)ₛOH, wherein s is 2 and R₁₀COOH, wherein R₁₀ is CH=CH; n is 2; q is 1; and r is 2.

In another preferred embodiment, R₁ is C₃ alkyl; R₅ is CH₃ (CH₂)ₚ wherein p is 1; R₆ is H; R₇ is selected from the group consisting of R₉COOH, wherein R₉ is CH=CH and (CH₂)ₜOH, wherein t is 2; R₈ is NH₂; f is 0; n is 2; q is 1; and r is 2.

The compound (I) may form pharmaceutically acceptable salts with both organic and inorganic acid and bases. Examples of suitable acids for salt formation are hydrochloric, sulfuric, phosphoric, acetic, citric, oxalic, malonic, salicylic, ascorbic, maleic, methanesulfonic, and the like. Any of the amine acid addition salts may also be used. The salts are prepared by contacting the free base form of the compound with an appropriate amount of the desired acid in a manner known to one skilled in the art. Examples of suitable bases for salt formation are sodium hydroxide, sodium carbonate, sodium bicarbonate, potassium hydroxide, calcium hydroxide, ammonia, organic amines, and the like. The salts may be prepared by contacting the free acid form of the compound with an appropriate amount of the desired base in a manner known to one skilled in the art.

The invention provides A₁ adenosine receptor antagonist compounds with radioactive or non-radioactive labels. Such labelled compounds are useful as assay-type probes or conjugates, and may be used to obtain quantitative binding measurements of the A₁ adenosine receptor antagonist compounds. For the purposes of the invention, "assay-type probes" refers to those materials which are useful for enhancing the selectivity of the quantitative analysis of the A₁ adenosine receptor compounds of the invention. Examples of such assay-type probes are described in U.S. Patent No. 5,248,770 to Jacobson et al., the disclosure of which is incorporated herein by reference in its entirety. The probes are highly useful in that they have little adverse effect on the affinity of the compounds of the present invention. Radioactive markers include, but are not limited to, an electric spin marker, a ¹⁹F NMR probe, a radioactive ¹⁸F isotope marker, a radioactive iodine marker (e.g., ¹²⁵I), a radioactive ³H marker, tritium, and a complex of a metal atom or a metal ion and a chelating agent. An exemplary metal ion is a radioactive isotope of technetium or indium. An exemplary chelating agent is diethylene pentacetic anhydride.

Various non-radioactive materials may be used in labelling the present A₁ adenosine receptor compounds. Numerous examples are presented in U.S. Patent No. 5,248,770 to Jacobson et al. Biotin is used as a common non-radioactive label for such probes, as described in R.W. Old et al. *Principals of Gene Manipulation,* **4th ed:** 328-331 (1989). To facilitate labelling the compounds with biotin or any other appropriate material, a spacer component may be added to the compound according to an accepted method. Such a method is described in the Jacobson et al. '770 patent. Exemplary spacer components include, but are not limited to, an oligopeptide, triglycidyl, and N-hydroxysuccinimide ester.

Biotin may be bonded to any suitable linkage provided by substituents on the compound structure in accordance with any accepted and suitable technique. For example, referring to compound (I) as defined herein, biotin may be bonded to the hydroxy group on R₆ when the compound contains (CH₂)ₘOH at R₆ with m defined herein; to the amino group present on either of R₇ or R₈ when (CH₂)_{f}NH₂ is contained at the R₈ position, wherein f is defined herein; to the hydroxyl group present as R₇ or R₈; or to the carboxyl group present when R₇ and R₈ are R₉COOH or R₁₀COOH respectively, with R₉ and R₁₀ defined herein. Additionally, the biotin may be bonded to a hydroxyl group present on R₈, when R₈ is (CH₂)ₛOH with s being defined herein. Biotin may also be bonded to R₇, when R₇ is (CH₂)ₜOH with t being defined herein. The biotin-labeled probes may be detected through appropriate and known analytical techniques.

Fluorescent dyes may also be employed as a non-radioactive labels and are applied to appropriate locations on the compounds disclosed above. Such dyes include, but are not limited to, tetramethylrhodamine, fluorescein isothiocyanate, and mixtures thereof. Other non-radioactive materials include for example, nitrobenzoxadiazole; 2,2,6,6-tetramethylpiperindinyloxy-4-isothiocyanate; and mixtures thereof.

The following example is illustrative of the synthesis of A₁ Adenosine Receptor Antagonists useful in the present invention.

### Example

### Synthesis of A₁ Adenosine Receptor Antagonists

A₁ adenosine receptor antagonists useful in the present invention may be synthesized according to the process illustrated below:

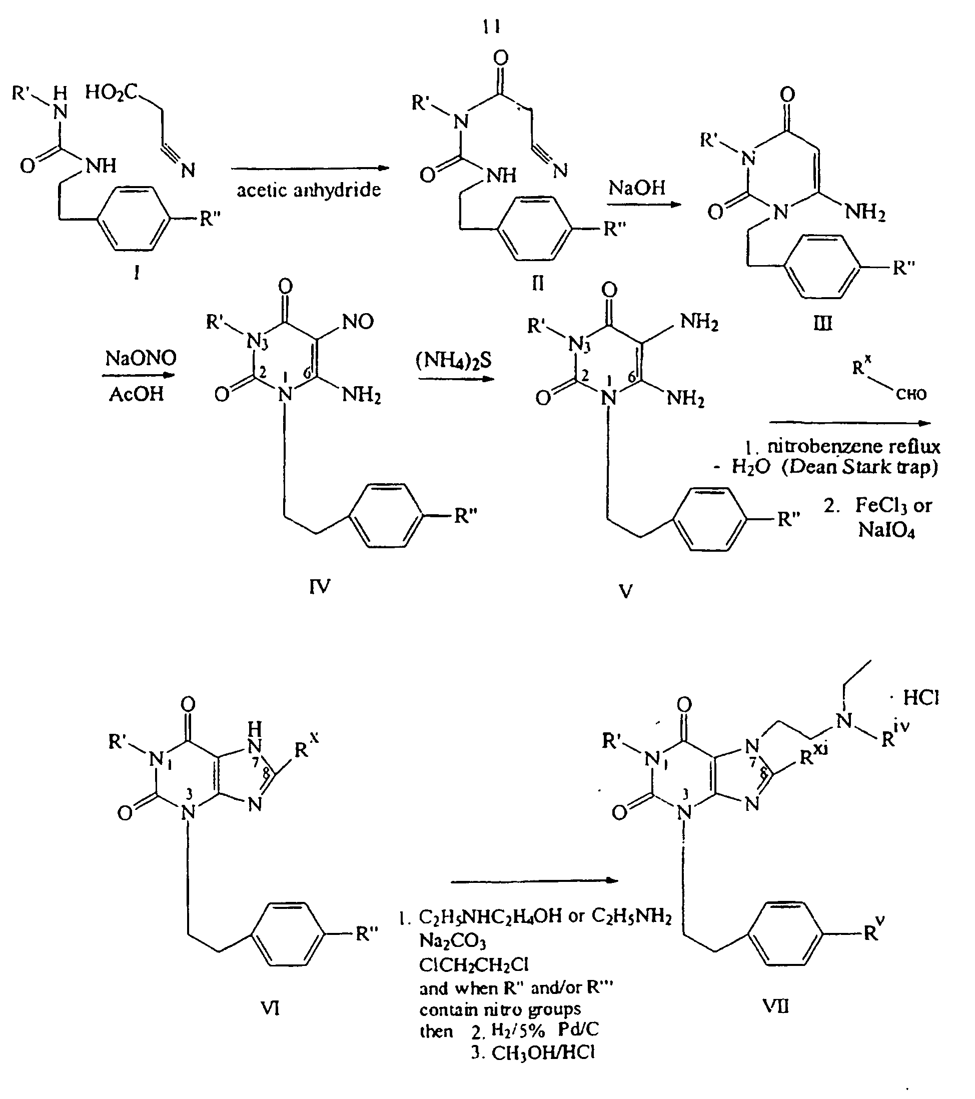

In the above reaction pathway, R' may be C₁-C₈ allcyl; R" may be selected from the group consisting of H, OH, (CH₃)ₑNO₂ wherein e is selected from the group consisting of 0 and an integer ranging from 1 to 8; (CH₂)ₛOH, wherein s is an integer ranging from 1 to 8; and R₁₀COOH, wherein R₁₀ is an alkylene or alkenylene group having 1 to 8 carbon atom; R^{x} is:

(CH₂)_{q}C₆H₄R'''

wherein R''' may be selected from the group consisting of H, OH, NO₂ R₉COOH, wherein R₉ is an alkylene or alkenylene group having 1 to 8 carbon atoms, and (CH₂)ₜOH, wherein t is an integer ranging from 1 to 8; and R^{iv} may be selected from the group consisting of H, (CH₂)ₘH, and (CH₂)ₘOH, wherein m is an integer ranging from 1 to 8. As identified in formula (VII), R^{v} may be selected from the group consisting of H; OH; (CH₂)_{f}NH₂ wherein f is selected from the group consisting of 0 and an integer ranging from 1 to 8; (CH₂)ₛOH, wherein s is an integer ranging from 1 to 8; and R₁₀COOH, wherein R₁₀ is an alkylene or alkenylene group having 1 to 8 carbon atoms. R^{xi} is:

(CH₂)_{q}C₆H₄R^{vi}

wherein R^{vi} may be selected from the group consisting of H, OH, NH₂, R₉COOH, wherein R₉ is an alkylene or alkenylene group having 1 to 8 carbon atoms, and (CH₂)ₜOH, wherein t is an integer ranging from 1 to 8. In general, the above synthesis steps may be carried out at standard temperature and pressure conditions, optionally under reflux. An exception to this pertains to the reaction involving intermediates (VI) and (VII) which is preferably performed at a temperature ranging from about 25EC to about 50EC and at atmospheric pressure. In the reaction step involving intermediate (V) becoming intermediate (VI), it is desired to employ an oxidation step with FeCl₃ or NalO₄ subsequent to the nitrobenzene reflux. Moreover, it should be noted that when R" and/or R"' contain nitro groups, a reaction step which involves applying H₂ over a Pd/C catalyst is employed prior to the reaction with CH₃OH/HCl. The resulting product (VII) may be further processed and purified according to accepted procedures. an integer ranging from 1 to 8. R₁₁ is selected from the group consisting of -CH₂COOH and -CH₂-CONH(CH₂)_{w}NHZ, wherein w is an integer ranging from 1 to 2 and Z is selected from the group consisting of hydrogen and acetate. In general, the above synthesis steps may be carried out at standard temperature and pressure conditions, optionally under reflux. An exception to this pertains to the reaction involving intermediates (V1) and (VII) which is preferably performed at a temperature ranging from about 25°C to about 50°C and at atmospheric pressure. In the reaction step involving intermediate (V) becoming intermediate (VI), it is desired to employ an oxidation step with FeCl₃ or NalO₄ subsequent to the nitrobenzene reflux. Moreover, it should be noted that when R" and/or R''' contain nitro groups, a reaction step which involves applying H₂ over a Pd/C catalyst is employed prior to the reaction with CH₃OH/HCl. The resulting product (VII) may be further processed and purified according to accepted procedures.

## Claims

1. An assay-type probe comprising
(1) a compound of the formula: 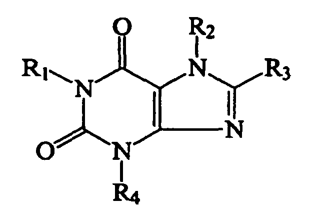
wherein R₁ is C₁-C₈ alkyl;
R₂ is of the formula: 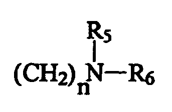
wherein n is an integer ranging from 1 to 8; R₅ is H or CH₃(CH₂)ₚ, wherein p is an integer ranging from 1 to 7; and R₆ is H; (CH₂)ₘH; or (CH₂)ₘOH, wherein m is an integer ranging from 1 to 8;
R₃ is
-(CH₂)_{q}C₆H₄-R₇
wherein q is an integer ranging from 1 to 8; wherein R₇ is selected from H, OH, NH₂, R₉COOH, wherein R₉ is an alkylene or alkenylene group having 1 to 8 carbon atoms, and (CH₂)ₜOH, wherein t is an integer ranging from 1 to 8; and
R₄ is of the formula: 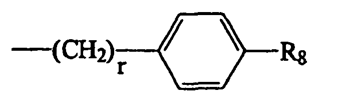
wherein R₈ is selected from H; OH; (CH₂)_{f}NH₂ wherein f is selected from 0 and an integer ranging from 1 to 8; (CH₂)ₛOH, wherein s is an integer ranging from 1 to 8; and R₁₀COOH, wherein R₁₀ is an alkylene or alkenylene group having 1 to 8 carbon atoms; and r is an integer ranging from 1 to 8, or a pharmaceutically acceptable salt thereof, and
(2) a radioactive or non-radioactive material,
wherein said assay-type probe is labelled with said radioactive or non-radioactive material, wherein when said compound is labelled with a non-radioactive material, a spacer component is present thereon wherein the spacer component has a functionality which bonds to the amine, hydroxyl or carboxy functionality present on the R₆, R₇ or R₈ substituent of said compound.

2. The assay-type probe according to Claim 1, wherein said non-radioactive material is a fluorescent dye or biotin.

3. The assay-type probe according to Claim 2 wherein said fluorescent dye is selected from tetramethylrhodamine, fluorescein isothiocyanate, and mixtures thereof.

4. The assay-type probe according to Claim 1, wherein said non-radioactive material is selected from nitrobenzoxadiazole, 2,2,6,6-tetramethylpiperidinyloxy-4-isothiocyanate, and mixtures thereof.

5. The assay-type probe according to Claim 1, wherein said radioactive material is a radioactive isotope selected from an electric spin marker, a ¹⁸F isotope marker, a radioactive ³H marker, a ¹⁹F probe, tritium, a complex of a metal atom, a complex of a metal ion, a chelating agent, and ¹²⁵I.

6. The assay-type probe according to claim 5 wherein said metal ion is a radioactive isotope of technetium or indium.

7. The assay-type probe according to claim 5 wherein said chelating agent is diethylene pentacetic anhydride.

8. The assay-type probe according to any preceding Claim, wherein R₁ is C₃ alkyl; R₃ is:
-(CH₂)_{q}C₆H₄-R₇
R₅ is CH₃(CH₂)ₚ wherein p is 1; R₆ is (CH₂)ₘOH wherein m is 2; R₇ is H; R₈ is NH₂; f is 0; n is 2; q is 1; and r is 2.

9. The assay-type probe according to any one of Claims 1 to 7, wherein R₁ is C₃ alkyl; R₃ is:
-(CH₂)_{q}C₆H₄-R₇
R₅ is CH₃(CH₂)ₚ wherein p is 1; R₆ is H; R₇ is NH₂; R₈ is NH₂; f is 0; n is 2; q is 1; and r is 2.

10. The assay-type probe according to any one of Claims 1 to 7, wherein R₁ is C₃ allcyl; R₃ is:
-(CH₂)_{q}C₆H₄-R₇
R₅ is CH₃(CH₂)ₚ wherein p is 1; R₆ is H; R₇ is H; R₈ is NH₂; f is 0; n is 2; q is 1; and ris2.

11. The assay-type probe according to any one of Claims 1 to 7, wherein R₁ is C₃ alkyl; R₃ is:
-(CH₂)_{q}C₆H₄-R₇
R₅ is CH₃(CH₂)ₚ wherein p is 1; R₆ is H; R₇ is H; R₈ is selected from the group consisting of (CH₂)ₛOH wherein s is 2 and R₁₀COOH, wherein R₁₀ is CH=CH; n is 2; q is 1; and r is 2.

12. The assay-type probe according to any one of Claims 1 to 7, wherein R₁ is C₃ alkyl; R₃ is:
-(CH₂)_{q}C₆H₄-R₇
R₅ is CH₃(CH₂)ₚ wherein p is 1; R₆ is H; R₇ is selected from the group consisting of H, OH, NH₂, (CH₂)ₜOH wherein t is 2 and R₉COOH, wherein R₉ is CH=CH; R₈ is NH₂; f is 0; n is 2; q is 1; and r is 2.

13. The assay-type probe according to Claim 12 wherein R₁ is C₃ alkyl; R₅ is CH₃(CH₂)ₚ wherein p is 1; R₇ is H; R₈ is NH₂; n is 2; q is 1; r is 2; and R₆ is (CH₂)ₘOH wherein m is 2; wherein said non-radioactive material is biotin bonded to the hydroxyl group present on R₆.

14. The assay-type probe according to Claim 12 wherein R₁ is C₃ alkyl; R₅ is CH₃(CH₂)ₚ wherein p is 1; R₆ is H; R₇ is NH₂; n is 2; q is 1; r is 2; and R₈ is NH₂; wherein said non-radioactive material is biotin bonded to the amino group present on R₈.

15. The assay-type probe according to Claim 12 wherein R₁ is C₃ alkyl; R₅ is CH₃(CH₂)ₚ wherein p is 1; R₆ is H; R₇ is H; n is 2; q is 1; r is 2; and R₈ is R₁₀COOH, wherein R₁₀ is an alkylene or alkenylene group having 1 to 8 carbon atoms; wherein said non- radioactive material is biotin bonded to the carboxyl group present on R₈.

16. The assay-type probe according to any one of Claims 1 to 7, wherein R₁ is C₃ alkyl; R₅ is CH₃(CH₂)ₚ wherein p is 1; R₆ is (CH₂)ₘOH wherein m is 2; R₇ is H; R₈ is NH₂; n is 2; q is 1; and r is 2.

17. The assay-type probe according to any one of Claims 1 to 7, wherein R₁ is C₃ alkyl; R₅ is CH₃(CH₂)ₚ wherein p is 1; R₆ is H; R₇ is NH₂; R₈ is NH₂; n is 2; q is 1; and r is 2.

18. The assay-type probe according to any one of Claims 1 to 7, wherein R₁ is C₃ alkyl; R₅ is CH₃(CH₂)ₚ wherein p is 1; R₆ is H; R₇ is H; R₈ is NH₂; n is 2; q is 1; and r is 2.

19. The assay-type probe according to any one of Claims 1 to 7, wherein R₁ is C₃ alkyl; R₅ is CH₃(CH₂)ₚ wherein p is 1; R₆ is H; R₇ is H; R₈ is selected from the group consisting of (CH₂)ₛOH, wherein s is 2 and R₁₀COOH, wherein R₁₀ is CH=CH; n is 2; q is 1; and r is 2.

20. The assay-type probe according to any one of Claims 1 to 7, wherein R₁ is C₃ alkyl; R₅ is CH₃(CH₂)ₚ wherein p is 1; R₆ is H; R₇ is selected from the group consisting of R₉COOH, wherein R₉ is CH=CH and (CH₂)ₜOH, wherein t is 2; R₈ is NH₂; n is 2; q is 1; and r is 2.

21. The assay-type probe according to claim 2, wherein said assay-type probe is labelled with biotin.

22. The assay-type probe according to claim 21, wherein R₁ is C₃ alkyl; R₅ is CH₃(CH₂)ₚ wherein p is 1; R₇ is H; R₈ is NH₂; n is 2; q is 1; r is 2; and R₆ is (CH₂)ₘOH wherein m is 2;
wherein the biotin is bonded to the hydroxyl group present on R₆.

23. The assay-type probe according to claim 21, wherein R₁ is C₃ alkyl; R₅ is CH₃(CH₂)ₚ wherein p is 1; R₆ is H; R₇ is NH₂; n is 2; q is 1; r is 2; and R₈ is NH₂;
wherein the biotin is bonded to the amino group present on R₈.

24. The assay-type probe according to claim 21, wherein R₁ is C₃ alkyl; R₅ is CH₃(CH₂)ₚ wherein p is 1; R₆ is H; R₇ is H; n is 2; q is 1; r is 2; and R₈ is R₁₀COOH, wherein R₁₀ is an alkylene or alkenylene group having 1 to 8 carbon atoms;
wherein the biotin is bonded to the carboxyl group present on R₈.

25. The assay-type probe according to claim 21, wherein R₁ is C₃ alkyl; R₅ is CH₃(CH₂)ₚ wherein p is 1; R₇ is OH; R₈ is NH₂; n is 2; q is 1; r is 2; and R₆ is (CH₂)ₘOH wherein m is 2;
wherein the biotin is bonded to the hydroxyl group present on R₇.

26. The assay-type probe according to claim 21, wherein R₁ is C₃ alkyl; R₅ is CH₃(CH₂)ₚ wherein p is 1; R₇ is NH₂; R₈ is NH₂; n is 2; q is 1; r is 2; and R₆ is (CH₂)ₘOH wherein m is 2;
wherein the biotin is bonded to the amino group present on R₇.

27. The assay-type probe according to claim 21, wherein R₁ is C₃ alkyl; R₅ is CH₃(CH₂)ₚ wherein p is 1; R₇ is R₉COOH; R₈ is NH₂; n is 2; q is 1; r is 2; and R₆ is (CH₂)ₘOH wherein m is 2;
wherein the biotin is bonded to the carboxyl group present on R₇.

28. The assay-type probe according to claim 21, wherein R₁ is C₃ alkyl; R₅ is CH₃(CH₂)ₚ wherein p is 1; R₇ is (CH₂)tOH wherein t is 2; R₈ is NH₂; n is 2; q is 1; r is 2; and R₆ is (CH₂)ₘOH wherein m is 2;
wherein the biotin is bonded to the hydroxyl group present on R₇.

## Patentansprüche

1. Assay-typische Sonde umfassend
(1) eine Verbindung der Formel: 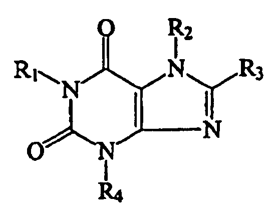
worin R₁ C₁-C₈-Alkyl ist;
R₂ die folgende Formel besitzt: 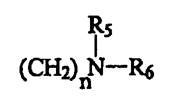
worin n eine ganze Zahl im Bereich von 1 bis 8 ist; R₅ H oder CH₃(CH₂)ₚ ist, worin p eine ganze Zahl im Bereich von 1 bis 7 ist; und R₆ H, (CH₂)ₘH oder (CH₂)ₘOH ist, worin m eine ganze Zahl im Bereich von 1 bis 8 ist;
R₃
-(CH₂)_{q}C₆H₄-R₇
ist, worin q eine ganze Zahl im Bereich von 1 bis 8 ist; worin R₇ ausgewählt ist aus H, OH, NH₂, R₉COOH, worin R₉ eine Alkylen- oder Alkenylengruppe ist, die 1 bis 8 Kohlenstoffatome besitzt, und (CH₂)ₜOH, worin t eine ganze Zahl im Bereich von 1 bis 8 ist; und
R₄ die folgende Formel besitzt: 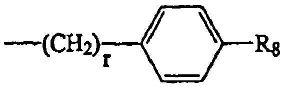
worin R₈ ausgewählt ist aus H, OH, (CH₂)_{f}NH₂, worin f ausgewählt ist aus 0 und einer ganzen Zahl im Bereich von 1 bis 8; (CH₂)ₛOH, worin s eine ganze Zahl im Bereich von 1 bis 8 ist; und R₁₀COOH, worin R₁₀ eine Alkylen- oder Alkenylengruppe ist, die 1 bis 8 Kohlenstoffatome besitzt; und r eine ganze Zahl im Bereich von 1 bis 8 ist, oder eines ihrer pharmazeutisch verträglichen Salze, und
(2) ein radioaktives oder nicht-radioaktives Material,
worin die genannte Assay-typische Sonde mit dem genannten radioaktiven oder nicht-radioaktiven Material markiert ist, worin, wenn die genannte Verbindung mit einem nicht-radioaktiven Material markiert ist, eine Spacer-Komponente darin vorhanden ist, worin die Spacer-Komponente eine Funktionalität besitzt, welche an die Amin-, Hydroxyl- oder Carboxy-Funktionalität bindet, die an dem R₆-, R₇- oder R₈-Substituenten der genannten Verbindung vorliegt.

2. Assay-typische Sonde gemäß Anspruch 1, worin das genannte nicht-radioaktive Material ein Fluoreszenzfarbstoff oder Biotin ist.

3. Assay-typische Sonde gemäß Anspruch 2, worin der genannte Fluoreszenzfarbstoff ausgewählt ist aus Tetramethylrhodamin, Fluoresceinisothiocyanat und deren Mischungen.

4. Assay-typische Sonde gemäß Anspruch 1, worin das genannte nicht-radioaktive Material ausgewählt ist aus Nitrobenzoxadiazol, 2,2,6,6-Tetramethylpiperidinyloxy-4-isothiocyanat und deren Mischungen.

5. Assay-typische Sonde gemäß Anspruch 1, worin das genannte radioaktive Material ein radioaktives Isotop ist, ausgewählt aus einem elektrischen Spin-Marker, einem ¹⁸F-Isotopen-Marker, einem radioaktiven ³H-Marker, einer ¹⁹_{F-}Sonde, Tritium, einem Komplex eines Metallatoms, einem Komplex eines Metallions, einem Chelatbildner und ¹²⁵I.

6. Assay-typische Sonde gemäß Anspruch 5, worin das genannte Metallion ein radioaktives Isotop von Technetium oder Indium ist.

7. Assay-typische Sonde gemäß Anspruch 5, worin der genannte Chelatbildner Diethylenpentaessigsäureanhydrid ist.

8. Assay-typische Sonde gemäß irgendeinem vorangehenden Anspruch, worin R₁ C₃-Alkyl ist; R₃ ist:
-(CH₂)_{q}C₆H₄-R₇
R₅ CH₃(CH₂)ₚ ist, worin p 1 ist; R₆ (CH₂)ₘOH ist, worin m 2 ist; R₇ H ist; R₈ NH₂ ist; f 0 ist; n 2 ist; q 1 ist und r 2 ist.

9. Assay-typische Sonde gemäß irgendeinem der Ansprüche 1 bis 7, worin R₁ C₃-Alkyl ist, R₃ ist:
-(CH₂)_{q}C₆H₄-R₇
R₅ CH₃(CH₂)ₚ ist, worin p 1 ist; R₆ H ist; R₇ NH₂ ist; R₈ NH₂ ist; f 0 ist; n 2 ist; q 1 ist und r 2 ist.

10. Assay-typische Sonde gemäß irgendeinem der Ansprüche 1 bis 7, worin R₁ C₃-Alkyl ist; R₃ ist:
-(CH₂)_{q}C₆H₄-R₇
R₅ CH₃(CH₂)ₚ ist, worin p 1 ist; R₆ H ist; R₇ H ist; R₈ NH₂ ist; f 0 ist; n 2 ist; q 1 ist und r 2 ist.

11. Assay-typische Sonde gemäß irgendeinem der Ansprüche 1 bis 7, worin R₁ C₃-Alkyl ist; R₃ ist:
-(CH₂)_{q}C₆H₄-R₇
R₅ CH₃(CH₂)ₚ ist, worin p 1 ist; R₆ H ist; R₇ H ist; R₈ ausgewählt ist aus der Gruppe, bestehend aus (CH₂)ₛOH, worin s 2 ist, und R₁₀COOH, worin R₁₀ CH=CH ist; n 2 ist; q 1 ist und r 2 ist.

12. Assay-typische Sonde gemäß irgendeinem der Ansprüche 1 bis 7, worin R₁ C₃-Alkyl ist; R₃ ist:
-(CH₂)_{q}C₆H₄-R₇
R₅ CH₃(CH₂)ₚ ist, worin p 1 ist; R₆ H ist; R₇ ausgewählt ist aus der Gruppe, bestehend aus H, OH, NH₂, (CH₂)ₜOH, worin t 2 ist, und R₉COOH, worin R₉ CH=CH ist; R₈ NH₂ ist, f 0 ist; n 2 ist; q 1 ist und r 2 ist.

13. Assay-typische Sonde gemäß Anspruch 12, worin R₁ C₃₋Alkyl ist; R₅ CH₃(CH₂)ₚ ist, worin p 1 ist; R₇ H ist; R₈ NH₂ ist; n 2 ist; q 1 ist; r 2 ist und R₆ (CH₂)ₘOH ist, worin m 2 ist; worin das genannte nicht-radioaktive Material Biotin ist, das an die an R₆ vorliegende Hydroxylgruppe gebunden ist.

14. Assay-typische Sonde gemäß Anspruch 12, worin R₁ C₃₋Alkyl ist; R₅ CH₃(CH₂)ₚ ist, worin p 1 ist; R₆ H ist; R₇ NH₂ ist; n 2 ist; q 1 ist; r 2 ist und R₈ NH₂ ist; worin das genannte nicht-radioaktive Material Biotin ist, das an die an R₈ vorliegende Aminogruppe gebunden ist.

15. Assay-typische Sonde gemäß Anspruch 12, worin R₁ C₃₋Alkyl ist; R₅ CH₃(CH₂)ₚ ist, worin p 1 ist; R₆ H ist; R₇ H ist; n 2 ist; q 1 ist; r 2 ist und R₈ R₁₀COOH ist, worin R₁₀ eine Alkylen- oder Alkenylengruppe ist, die 1 bis 8 Kohlenstoffatome besitzt; worin das genannte nicht-radioaktive Material Biotin ist, das an die an R₈ vorliegende Carboxylgruppe gebunden ist.

16. Assay-typische Sonde gemäß irgendeinem der Ansprüche 1 bis 7, worin R₁ C₃-Alkyl ist; R₅ CH₃(CH₂)ₚ ist, worin p 1 ist; R₆ (CH₂)ₘOH ist, worin m 2 ist; R₇ H ist; R₈ NH₂ ist; n 2 ist; q 1 ist und r 2 ist.

17. Assay-typische Sonde gemäß irgendeinem der Ansprüche 1 bis 7, worin R₁ C₃-Alkyl ist; R₅ CH₃(CH₂)ₚ ist, worin p 1 ist; R₆ H ist; R₇ NH₂ ist; R₈ NH₂ ist; n 2 ist; q 1 ist und r 2 ist.

18. Assay-typische Sonde gemäß irgendeinem der Ansprüche 1 bis 7, worin R₁ C₃-Alkyl ist; R₅ CH₃(CH₂)ₚ ist, worin p 1 ist; R₆ H ist; R₇ H ist; R₈ NH₂ ist; n 2 ist; q 1 ist und r 2 ist.

19. Assay-typische Sonde gemäß irgendeinem der Ansprüche 1 bis 7, worin R₁ C₃-Alkyl ist; R₅ CH₃(CH₂)ₚ ist, worin p 1 ist; R₆ H ist; R₇ H ist; R₈ ausgewählt ist aus der Gruppe, bestehend aus (CH₂)ₛOH, worin s 2 ist, und R₁₀COOH, worin R₁₀ CH=CH ist; n 2 ist; q 1 ist und r 2 ist.

20. Assay-typische Sonde gemäß irgendeinem der Ansprüche 1 bis 7, worin R₁ C₃-Alkyl ist; R₅ CH₃(CH₂)ₚ ist, worin p 1 ist; R₆ H ist; R₇ ausgewählt ist aus der Gruppe, bestehend aus R₉COOH, worin R₉ CH=CH ist, und (CH₂)ₜOH, worin t 2 ist; R₈ NH₂ ist; n 2 ist; q 1 ist und r 2 ist.

21. Assay-typische Sonde gemäß Anspruch 2, worin die genannte Assay-typische Sonde mit Biotin markiert ist.

22. Assay-typische Sonde gemäß Anspruch 21, worin R₁ C₃₋Alkyl ist; R₅ CH₃(CH₂)ₚ ist, worin p 1 ist; R₇ H ist; R₈ NH₂ ist; n 2 ist; q 1 ist; r 2 ist und R₆ (CH₂)ₘOH ist, worin m 2 ist;
worin das Biotin an die an R₆ vorliegende Hydroxylgruppe gebunden ist.

23. Assay-typische Sonde gemäß Anspruch 21, worin R₁ C₃₋Alkyl ist; R₅ CH₃(CH₂)ₚ ist, worin p 1 ist; R₆ H ist; R₇ NH₂ ist; n 2 ist; q 1 ist; r 2 ist und R₈ NH₂ ist;
worin das Biotin an die an R₈ vorliegende Aminogruppe gebunden ist.

24. Assay-typische Sonde gemäß Anspruch 21, worin R₁ C₃₋Alkyl ist; R₅ CH₃(CH₂)ₚ ist, worin p 1 ist; R₆ H ist; R₇ H ist; n 2 ist; q 1 ist; r 2 ist und R₈ R₁₀COOH ist, worin R₁₀ eine Alkylen- oder Alkenylengruppe ist, die 1 bis 8 Kohlenstoffatome besitzt;
worin das Biotin an die an R₈ vorliegende Carboxylgruppe gebunden ist.

25. Assay-typische Sonde gemäß Anspruch 21, worin R₁ C₃₋Alkyl ist; R₅ CH₃(CH₂)ₚ ist, worin p 1 ist; R₇ OH ist; R₈ NH₂ ist; n 2 ist; q 1 ist; r 2 ist und R₆ (CH₂)ₘOH ist, worin m 2 ist;
worin das Biotin an die an R₇ vorliegende Hydroxylgruppe gebunden ist.

26. Assay-typische Sonde gemäß Anspruch 21, worin R₁ C₃₋Alkyl ist; R₅ CH₃(CH₂)ₚ ist, worin p 1 ist; R₇ NH₂ ist; R₈ NH₂ ist; n 2 ist; q 1 ist; r 2 ist und R₆ (CH₂)ₘOH ist, worin m 2 ist;
worin das Biotin an die an R₇ vorliegende Aminogruppe gebunden ist.

27. Assay-typische Sonde gemäß Anspruch 21, worin R₁ C₃₋Alkyl ist; R₅ CH₃(CH₂)ₚ ist, worin p 1 ist; R₇ R₉COOH ist; R₈ NH₂ ist; n 2 ist; q 1 ist; r 2 ist und
R₆ (CH₂)ₘOH ist, worin m 2 ist;
worin das Biotin an die an R₇ vorliegende Carboxylgruppe gebunden ist.

28. Assay-typische Sonde gemäß Anspruch 21, worin R₁ C₃₋Alkyl ist; R₅ CH₃(CH₂)ₚ ist, worin p 1 ist; R₇ (CH₂)ₜOH ist, worin t 2 ist; R₈ NH₂ ist; n 2 ist; q 1 ist; r 2 ist und R₆ (CH₂)ₘOH ist, worin m 2 ist;
worin das Biotin an die an R₇ vorliegende Hydroxylgruppe gebunden ist.

## Revendications

1. Sonde du type analyse comprenant
(1) un composé de formule: 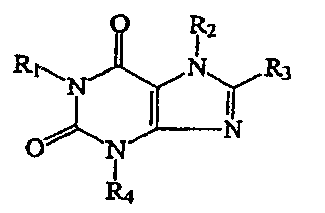
où R₁ est un alkyle C₁-C₈ ;
R₂ est de formule : 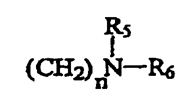
où n est un entier allant de 1 à 8 ; R₅ est H ou CH₃(CH₂)ₚ, où p est un entier allant de 1 à 7 ; et R₆ est H ; (CH₂)ₘH ; ou (CH₂)ₘOH, où m est un entier allant de 1 à 8 ;
R₃ est
-(CH₂)_{q}C₆H₄-R₇
Où q est un entier allant de 1 à 8 ; tandis que R₇ est choisi parmi H, OH, NH₂, R₉COOH, où R₉ est un groupe alkylène ou alkénylène ayant 1 à 8 atomes de carbone, et (CH₂)₁OH, où t est un entier allant de 1 à 8 ; et
R₄ est de la formule : 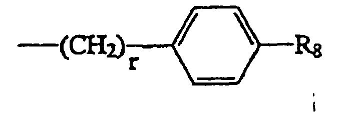
Où R₈ est choisi parmi H ; OH ; (CH₂)_{f}NH₂ où f est choisi entre 0 et un entier allant de 1 à 8 ; (CH₂)ₛOH, où s est un entier allant de 1 à 8 ; et R₁₀COOH, où R₁₀ est un alkylène ou alkénylène groupe ayant 1 à 8 atomes de carbone ; et r est un entier allant de 1 à 8, ou un sel de celui-ci acceptable sur le plan pharmaceutique, et
(2) un matériau radioactif ou non radioactif,
**caractérisée en ce que** ladite sonde du type analyse est marquée avec ledit matériau radioactif ou non radioactif, et **en ce que** ledit composé est marqué avec un matériau non radioactif, un composant espaceur étant disposé sur celui-ci tandis que le composant espaceur présente une fonctionnalité de liaison à l'amine, hydroxyle ou carboxy fonctionnellement présent sur le substituant R₆, R₇ ou R₈ dudit composé.

2. Sonde du type analyse selon la revendication 1, **caractérisée en ce que** ledit matériau non radioactif est un colorant fluorescent ou de la biotine.

3. Sonde du type analyse selon la revendication 2, **caractérisée en ce que** ledit colorant fluorescent est choisi parmi tétraméthylrhodamine, fluorescéine isothiocyanate, et des mélanges de ces derniers.

4. Sonde du type analyse selon la revendication 1, **caractérisée en ce que** ledit matériau non radioactif est choisi parmi nitrobenzoxadiazole, 2, 2, 6, 6-tétraméthylpipéridinyloxy-4-isothiocyanate, et des mélanges de celui-ci.

5. Sonde du type analyse selon la revendication 1, **caractérisée en ce que** ledit matériau radioactif est un isotope radioactif choisi parmi un marqueur à spin électrique, un isotope ¹⁸F marqueur, un marqueur radioactif ³H, une sonde ¹⁹F, du tritium, un complexe d'un atome métallique, un complexe d'un ion métallique, un agent chelatant, et ¹²⁵I.

6. Sonde du type analyse selon la revendication 5, **caractérisée en ce que** ledit ion métallique est un isotope radioactif de technétium ou indium.

7. Sonde du type analyse selon la revendication 5, **caractérisée en ce que** ledit agent chelatant est un anhydride pentacétique diéthylène.

8. Sonde du type analyse selon l'une des revendications précédentes, **caractérisée en ce que** R₁ est C₃ alkyle ; R₃ est
-(CH₂)_{q}C₆H₄-R₇
R₅ est CH₃(CH₂)ₚ où p est 1 ; R₆ est (CH₂)ₘOH où m est 2 ; R₇ est H ; R₈ est NH2 ; f est 0 ; n est 2 ; q est 1 ; et r est 2.

9. Sonde du type analyse selon l'une des revendications 1 à 7, **caractérisée en ce que** R₁ est alkyle C₃ ; R₃ est :
-(CH₂)_{q}C₆H₄-R₇
R₅ est CH₃(CH₂)ₚ où p est 1 ; R₆ est H ; R₇ est NH₂ ; R₈ est NH₂ ; f est 0 ; n est 2 ; q est 1 ; et r est 2.

10. Sonde du type analyse selon l'une des revendications 1 à 7, **caractérisée en ce que** R₁ est alkyle C₃ ; R₃ est :
-(CH₂)_{q}C₆H₄-R₇
R₅ est CH₃(CH₂)ₚ où p est 1 ; R₆ est H ; R₇ est H ; R₈ est NH₂ ; f est 0 ; n est 2 ; q est 1 ; et r est 2.

11. Sonde du type analyse selon l'une des revendications 1 à 7, **caractérisée en ce que** R₁ est alkyle C₃ ; R₃ est :
-(CH₂)_{q}C₆H₄-R₇
R₅ est CH₃(CH₂)ₚ où p est 1 ; R₆ est H ; R₇ est H ; R₈ est sélectionné parmi le groupe consistant de (CH₂)ₛOH où s est 2 et R₁₀COOH, où R₁₀ est CH=CH ; n est 2 ; q est 1 ; et r est 2.

12. Sonde du type analyse selon l'une des revendications 1 à 7, **caractérisée en ce que** R₁ est alkyle C₃ ; R₃ est :
-(CH₂)_{q}C₆H₄-R₇
R₅ est CH₃(CH₂)ₚ où p est 1 ; R₆ est H ; R₇ est sélectionné parmi le groupe consistant de H, OH, NH₂, (CH₂)ₜOH où t est 2 et R₉COOH, où R₉ est CH=CH ; R₈ est NH₂ ; f est 0 ; n est 2 ; q est 1 ; et r est 2.

13. Sonde du type analyse selon la revendication 12, **caractérisée en ce que** R₁ est alkyle C₃ ; R₅ est CH₃(CH₂)ₚ où p est 1 ; R₇ est H ; R₈ est NH₂ ; n est 2 ; q est 1 ; r est 2 ; et R₆ est (CH₂)ₘOH où m est 2 ; tandis que ledit matériau non radioactif est de la biotine liée à un groupe hydroxyle présent sur R₆.

14. Sonde du type analyse selon la revendication 12, **caractérisée en ce que** R₁ est alkyle C₃ ; R₅ est CH₃(CH₂)ₚ où p est 1 ; R₆ est H ; R₇ est NH₂ ; n est 2 ; q est 1 ; r est 2 ; et R₈ est NH₂ ; tandis que ledit matériau non radioactif est de la biotine liée à un groupe amino présent sur R₈.

15. Sonde du type analyse selon la revendication 12, **caractérisée en ce que** R₁ est alkyle C₃ ; R₅ est CH₃(CH₂)ₚ où p est 1 ; R₆ est H ; R₇ est H ; n est 2 ; q est 1 ; r est 2 ; et R₈ est R₁₀COOH, où R₁₀ est groupe alkylène ou alkénylène ayant 1 à 8 atomes de carbone ; tandis que ledit matériau non radioactif est de la biotine liée à un groupe carboxyle présent sur R₈.

16. Sonde du type analyse selon l'une des revendications 1 à 7, **caractérisée en ce que** R₁ est alkyle C₃; R₅ est CH₃(CH₂)ₚ où p est 1 ; R₆ est (CH₂)ₘOH où m est 2 ; R₇ est H ; R₈ est NH₂ ; n est 2 ; q est 1 ; et r est 2.

17. Sonde du type analyse selon l'une des revendications 1 à 7, **caractérisée en ce que** R₁ est alkyle C₃ ; R₅ est CH₃(CH₂)ₚ ; p est 1 ; R₆ est H ; R₇ est NH₂ ; R₈ est NH₂ ; n est 2 ; q est 1 ; et r est 2.

18. Sonde du type analyse selon l'une des revendications 1 à 7, **caractérisée en ce que** R₁ est alkyle C₃ ; R₅ est CH₃(CH₂)ₚ ; p est 1 ; R₆ est H ; R₇ est H ; R₈ est NH₂ ; n est 2 ; q est 1 ; et r est 2.

19. Sonde du type analyse selon l'une des revendications 1 à 7, **caractérisée en ce que** R₁ est alkyle C₃ ; R₅ est CH₃(CH₂)ₚ où p est 1 ; R₆ est H ; R₇ est H ; R₈ est sélectionné parmi le groupe consistant en (CH₂)ₛOH où s est 2 et R₁₀COOH, où R₁₀ est CH=CH ; n est 2 ; q est 1 ; et r est 2.

20. Sonde du type analyse selon l'une des revendications 1 à 7, **caractérisée en ce que** R₁ est alkyle C₃ ; R₅ est CH₃(CH₂)ₚ où p est 1 ; R₆ est H ; R₇ est sélectionné parmi le groupe consistant en R₉COOH, où R₉ est CH=CH et (CH₂)ₜOH, où t est 2 ; R₈ est NH₂ ; n est 2 ; q est 1 ; et r est 2.

21. Sonde du type analyse selon la revendication 2, **caractérisée en ce que** ladite sonde du type analyse est marquée avec de la biotine.

22. Sonde du type analyse selon la revendication 21, **caractérisée en ce que** R₁ est alkyle C₃ ; R₅ est CH₃(CH₂)ₚ où p est 1 ; R₇ est H ; R₈ est NH₂ ; n est 2 ; q est 1 ; r est 2 ; et R₆ est (CH₂)ₘOH où m est 2 ; **caractérisée en ce que** la biotine est liée au groupe hydroxyle présent sur R₆.

23. Sonde du type analyse selon la revendication 21, **caractérisée en ce que** R₁ est alkyle C₃ ; R₅ est CH₃(CH₂)ₚ où p est 1 ; R₆ est H ; R₇ est NH₂ ; n est 2 ; q est 1 ; r est 2 ; et R₈ est NH₂ ;
**caractérisée en ce que** la biotine est liée au groupe amino présent sur R₈.

24. Sonde du type analyse selon la revendication 21, **caractérisée en ce que** R₁ est alkyle C₃ ; R₅ est CH₃(CH₂)ₚ où p est 1 ; R₆ est H ; R₇ est H; n est 2 ; q est 1 ; r est 2 ; et R₈ est R₁₀COOH, **caractérisée en ce que** R₁₀ est un groupe alkylène ou alkénylène ayant 1 à 8 atomes de carbone ; et **en ce que** la biotine est liée au groupe carboxyle présent sur R₈.

25. Sonde du type analyse selon la revendication 21, **caractérisée en ce que** R₁ est alkyle C₃ ; R₅ est CH₃(CH₂)ₚ où p est 1 ; R₇ est OH ; R₈ est NH₂ ; n est 2 ; q est 1 ; r est 2 ; et R₆ est (CH₂)ₘOH où m est 2 ; **caractérisée en ce que** la biotine est liée au groupe hydroxyle présent sur R₇.

26. Sonde du type analyse selon la revendication 21, **caractérisée en ce que** R₁ est alkyle C₃ ; R₅ est CH₃(CH₂)ₚ où p est 1 ; R₇ est NH₂ ; R₈ est NH₂ ; n est 2 ; q est 1 ; r est 2 ; et R₆ est (CH₂)ₘOH où m est 2 ; **caractérisée en ce que** la biotine est liée au groupe amino présent sur R₇.

27. Sonde du type analyse selon la revendication 21, **caractérisée en ce que** R₁ est alkyle C₃ ; R₅ est CH₃(CH₂)ₚ où p est 1 ; R₇ est R₉COOH ; R₈ est NH₂ ; n est 2 ; q est 1 ; r est 2 ; et R₆ est (CH₂)ₘOH où m est 2 ; **caractérisée en ce que** la biotine est liée au groupe carboxyle présent sur R₇.

28. Sonde du type analyse selon la revendication 21, **caractérisée en ce que** R₁ est alkyle C₃ ; R₅ est CH₃(CH₂)ₚ où p est 1 ; R₇ est (CH₂)ₜOH où t est 2 ; R₈ est NH₂ ; n est 2 ; q est 1 ; r est 2 ; et R₆ est (CH₂)ₘOH où m est 2 ; **caractérisée en ce que** la biotine est liée au groupe hydroxyle présent sur R₇.
